# EUROPEAN PATENT SPECIFICATION

(11) **EP 4 467 971 B1**
(45) Date of publication and mention of the grant of the patent: **09.12.2026**
(21) Application number: 23175381.5
(22) Date of filing: 25.05.2023
(51) Int. Cl.: G01N 27/12, G01N 33/00

(54) **GAS SENSOR FOR DETECTING VOLATILE ORGANIC COMPOUNDS AND METHOD FOR MANUFACTURING SAME**
GASSENSOR ZUR DETEKTION VON FLÜCHTIGEN ORGANISCHEN VERBINDUNGEN UND VERFAHREN ZUR HERSTELLUNG DAVON
CAPTEUR DE GAZ POUR DÉTECTER DES COMPOSÉS ORGANIQUES VOLATILS ET SON PROCÉDÉ DE FABRICATION

(43) Date of publication of application: 27.11.2024
(73) Proprietor: LG Electronics Inc., Yeongdeungpo-gu Seoul 07336 (KR)
(72) Inventor: TAKADA, Masaki, Seoul (KR)
(74) Representative: Vossius & Partner Patentanwälte Rechtsanwälte mbB

(56) References cited:
- CN-B- 102 680 540
- JP-A- 2007 139 669
- KR-B1- 102 181 598
- LING T R ET AL: "Influence of nano-scale dopants of Pt, CaO and SiO"2, on the alcohol sensing of SnO"2 thin films", SENSORS AND ACTUATORS B: CHEMICAL, ELSEVIER BV, NL, vol. 119, no. 2, 7 December 2006 (2006-12-07), pages 497 - 503, XP027971712, ISSN: 0925-4005, [retrieved on 20061207]
- MIN B K ET AL: "Undoped and 0.1wt.% Ca-doped Pt-catalyzed SnO"2 sensors for CH"4 detection", SENSORS AND ACTUATORS B: CHEMICAL, ELSEVIER BV, NL, vol. 108, no. 1-2, 22 July 2005 (2005-07-22), pages 119 - 124, XP004928440, ISSN: 0925-4005, DOI: 10.1016/J.SNB.2004.12.112

## Description

### Field of the Invention

The present disclosure relates generally to a gas sensor for detecting volatile organic compounds. More particularly, the present disclosure relates to a gas sensor capable of specifically detecting formaldehyde among volatile organic compounds and a method for manufacturing the same.

### Description of the Related Art

Physical disorders such as sore eyes, headaches, and allergies may occur indoors immediately after new construction or renovation. This is called a sick building syndrome, and the cause is known to be volatile organic compounds (VOC), primarily, formaldehyde.

Among volatile organic compounds, formaldehyde is contained in various adhesives, flooring materials, and particle board or plywood, and is released when these are used in indoor spaces, causing problems. It is impossible to completely avoid use of these things, and when volatile organic compounds including formaldehyde are generated therefrom, the volatile organic compounds have many adverse effects on indoor residents.

Looking at the effect of formaldehyde on the human body, when concentration of formaldehyde in the air is about 0.04ppm, nervous tissues begin to be stimulated, and sensitive children may develop atopic dermatitis. When the concentration of formaldehyde is 0.05 to 1.0ppm, it can be smelled, and the level of the concentration is an indoor standard in Canada and California. When concentration of formaldehyde in the air is 0.2ppm, eye irritation begins, and when the concentration is 0.25 to 0.33ppm, a respiratory failure may occur. The concentration of 0.5 ppm is the lowest level at which stimulation of the throat begins and is the highest level allowed by the Korean Society of Industrial Hygiene. When the concentration is 2.0 to 3.0ppm, pain in the eyes begins, and when the concentration is 4.0ppm, tearing may occur. When the concentration is 10.0 to 20.0ppm, intense tearing and difficulty in breathing may occur, and when the concentration is 30.0 ppm or more, acute poisoning may occur within minutes and death may occur from toxic emphysema.

For reference, in the indoor environmental standards set by the World Health Organization (WHO) and Japan, the concentration of formaldehyde in the air is 0.08ppm. In accordance with these standards, it is necessary to detect the concentration of formaldehyde in the air by using a gas sensor.

However, when formaldehyde is generated, other volatile organic compound (VOC) gases such as alcohol may exist. However, since general volatile organic compounds and formaldehyde have different effects on the human body, the specific and selective sensitivity of the gas sensor to formaldehyde is required.

A general gas sensor has similar sensitivity to most volatile organic compounds, and such an example can be seen in Japanese Patent No. 3854358. Here, electrodes made of gold (Au) are formed on a substrate, and tin oxide (SnO₂) to which at least one element of Ga, Y, La, and Nd is added is used as a sensing material between the electrodes.

However, such a gas sensor of a prior art has similar sensitivity to most volatile organic compounds. For example, when using a sensing material in which Pd or Gd is added to SiO₂, the sensitivity S of the gas sensor to 0.1ppm of formaldehyde is 20%, but the sensitivity S of the gas sensor to 1 ppm of volatile organic compounds also is 25%. The sensitivity is obtained by converting the percentage of a resistance change. Accordingly, the gas sensor of the prior art detects both formaldehyde and volatile organic compounds with high sensitivity, but cannot selectively detect only formaldehyde.

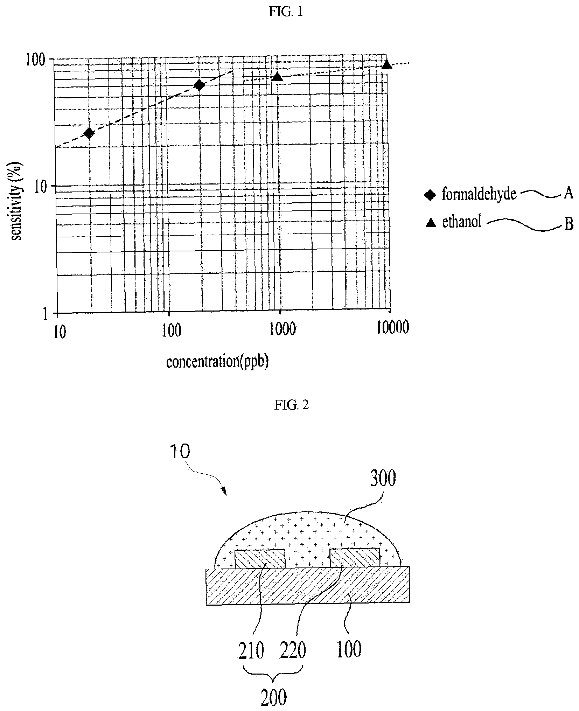

FIG. 1 illustrates the responsiveness of the gas sensor of the prior art. This is disposed in a paper titled "Study on a micro-gas sensor with SnO2-NiO sensitive film for indoor formaldehyde detection"(Sensors and Actuators B vol 132, (2008) p74~80). Here, the gas sensor is a MEMS structure using SnO₂-NiO as a sensing material and using Pt in making an electrode to detect formaldehyde.

However, the gas sensor in this paper has a sensitivity S of 0.06ppm of formaldehyde which is 1.6% and a sensitivity S of 1ppm of ethanol which is 0.1% or less. Furthermore, the sensitivity ratio of formaldehyde to ethanol is greater than 10, and the interference of the ethanol gas is suppressed. However, the sensitivity S of formaldehyde is 1.6%, which is very low.

In other words, the gas sensor is more sensitive to formaldehyde than volatile organic compounds including alcohol, but has a fundamental limitation in that the gas sensor cannot selectively detect formaldehyde.

Accordingly, the gas sensor of the prior art detects the presence of volatile organic compounds, but has a problem in that the gas sensor cannot provide information on whether formaldehyde is included in the volatile organic compounds and if included, how much formaldehyde is included therein.

JP 2007 139669 A, CN 102 680 540 B, Tzong-Rong Ling et al., "Influence of nano-scale dopants of Pt, CaO and SiO2, on the alcohol sensing of SnO2 thin films" in Sensors and Actuators B, Elsevier BV, NL, vol. 119, no. 2, 7 December 2006, Bong-Ki Min et al., "Undoped and 0.1 wt. % Ca-doped Pt-catalyzed Sn-O2 sensors for CH4 detection" in Sensors and Actuators B, Elsevier BV, NL, vol. 108, no. 1-2, 22 July 2005 and KR 102 181 598 B1 are documents of the related art.

### SUMMARY OF THE INVENTION

An object of the present invention is to provide a gas sensor with the highest sensitivity to formaldehyde among volatile organic compounds.

Another object of the present invention is to provide a gas sensor capable of detecting the presence or concentration of formaldehyde among volatile organic compounds.

Still another object of the present invention is to provide a gas sensor that maintains both high sensitivity and high selectivity.

These objects are achieved with the subject-matter of the independent claims. Preferred aspects are defined in the dependent claims.

The gas sensor of the present disclosure may have at least one of the following effects.

According to the present disclosure, formaldehyde gas at a low concentration of 10 ppb may be detected.

According to the present disclosure, the interference of other gases with formaldehyde may be avoided. Accordingly, only formaldehyde may be effectively detected.

In the gas sensor of the present disclosure, 1ppm of ethanol gas, which is a representative volatile organic compound, has sensitivity corresponding to 10ppb of formaldehyde or less. Accordingly, the gas sensor of the present disclosure may detect formaldehyde better than other volatile organic compounds, thereby having high selectivity to formaldehyde.

### BRIEF DESCRIPTION OF THE DRAWINGS

FIG. 1 is a graph illustrating that the selectivity of formaldehyde is lower than that of ethanol in a gas sensor of a prior art;
FIG. 2 is a schematic cross-sectional view illustrating the structure of a gas sensor of the present disclosure according to an embodiment;
FIG. 3 is a graph illustrating the sensitivity of the gas sensor of the present disclosure to formaldehyde, ethanol, and toluene;
FIGS. 4a and 4b are views illustrating gas detection performed by the gas sensor of the present disclosure;
FIG. 5 is a schematic cross-sectional view illustrating another embodiment of the gas sensor of the present disclosure;
FIG. 6 is a schematic cross-sectional view illustrating still another embodiment of the gas sensor of the present disclosure; and
FIG. 7 is a schematic cross-sectional view illustrating that a heater is added to the gas sensor in the embodiment illustrated in FIG. 2.

### DETAILED DESCRIPTION OF THE INVENTION

Hereinafter, embodiments disclosed in this specification will be described in detail with reference to the accompanying drawings. In this specification, the same or similar reference numerals are given to the same or similar components even in different embodiments, and the description thereof is replaced with the first description. Singular expressions used in this specification include plural expressions unless the context clearly dictates otherwise. In addition, in describing the embodiments disclosed in this specification, when it is determined that a detailed description of related known technologies may obscure the gist of the embodiments disclosed in this specification, the detailed description thereof will be omitted. In addition, it should be noted that the accompanying drawings are only for easy understanding of the embodiments disclosed in this specification, and should not be construed as limiting the technical idea disclosed in this specification by the accompanying drawings.

FIG. 2 schematically illustrates the structure of the gas sensor according to an embodiment of the present disclosure. The gas sensor 10 according to the embodiment of the present disclosure has a substrate 100 constituting a frame thereof. Electrodes 200 are located on the substrate 100. A sensing layer 300 is provided to connect each of the electrodes 200 to each other.

The substrate 100 is made of a non-conductive material such as silicon dioxide (SiO₂). The substrate 100 made of a resin-based material serves to support the electrode 200 and the sensing layer 300. The substrate 100 may be a plate having a predetermined shape. A glass substrate or an alumina substrate may be used as the substrate 100.

The electrode 200 may include at least one pair of a first electrode 210 and a second electrode 220. The first electrode 210 and the second electrode 220 may be electrically connected to the outside. The first electrode 210 and the second electrode 220 may be formed at a predetermined interval on the substrate 100, and an electric current may be transmitted between the first electrode 210 and the second electrode 220 through the sensing layer 300.

The gas sensor 10 of the present disclosure may detect formaldehyde by detecting an electric current flowing between the first electrode 210 and the second electrode 220, change in the electric current, or a change in electrical resistance or capacitance of the electrode 200.

The sensing layer 300 may be formed to cover the first electrode 210 and the second electrode 220. The sensing layer 300 may change the intensity of an electric current flowing between the first electrode 210 and the second electrode 220, or the capacitance or electrical resistance of the electrode 200. That is, a material forming the sensing layer 300 has inherent resistance, but the sensing layer 300 may have electrical properties changed when the sensing layer 300 contacts or is combined with formaldehyde.

For example, when a material of which the sensing layer 300 is formed comes into contact with or is combined with formaldehyde, the material may transfer electrons to the electrode 200 or receive electrons from the electrode 200 while causing an oxidation-reduction reaction with the formaldehyde. Alternatively, when a material of which the sensing layer 300 is formed comes into contact with or is combined with formaldehyde, the molecular arrangement structure of the sensing layer 300 may be changed due to the permeation of the formaldehyde, and thus the total charge capacity of the sensing layer 300 may be changed.

However, the sensing layer 300 may not react to other volatile organic compounds other than formaldehyde or may have a very low reactivity thereto compared to formaldehyde.

According to these characteristics, the gas sensor 10 of the present disclosure specifically detects formaldehyde. This is because the sensing layer 300 does not react or has very low reactivity to volatile organic compounds other than formaldehyde even when the formaldehyde exists along with the volatile organic compounds in the air or indoors.

The gas sensor 10 of the present disclosure specifically reacts to formaldehyde to detect formaldehyde, and may not react to other volatile organic compounds. Accordingly, hereinafter, the characteristics of the gas sensor 10 of the present disclosure not detecting other volatile organic compounds or having a low sensitivity to the other volatile organic compounds will be described.

In the gas sensor 10 of the present invention, materials of the electrode 200 and the sensing layer 300 are specified. That is, the materials of the electrode 200 and the sensing layer 300 are required to react specifically to formaldehyde or have a significantly greater sensitivity to formaldehyde than other volatile organic compounds.

A study found that when the material of the sensing layer 300 of the present disclosure is a compound of a tin oxide (SnO₂) with an oxide of an alkali metal element or an oxide of an alkaline earth metal element, the sensing layer 300 has a significantly greater sensitivity to formaldehyde than to other volatile organic compounds, or reacts specifically only to the formaldehyde.

Accordingly, as the material of the sensing layer 300 of the gas sensor 10 of the present disclosure, Mg-SnO₂ is selected.

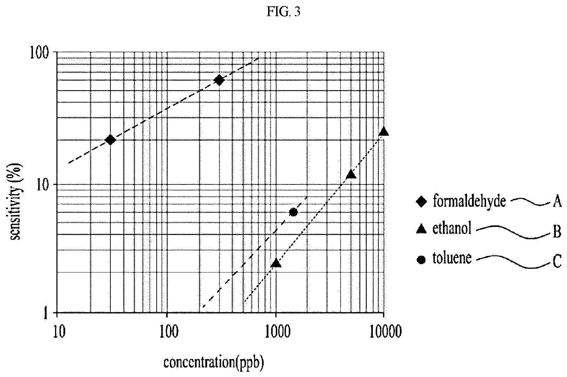

With the use of Mg-SnO₂ as the material of the sensing layer 300 of the present disclosure, the sensitivity of the sensing layer 300 to ethanol, toluene, and formaldehyde is shown graphically in FIG. 3. Referring to FIG. 3, it can be seen that the sensing layer 300 has a much higher sensitivity to formaldehyde A than to ethanol B and toluene C, which react most competitively with formaldehyde A among volatile organic compounds.

That is, it can be seen that the sensitivity of the gas sensor 10 of the present disclosure to formaldehyde A is between 20% and 70%, and ethanol B and toluene C are less than 10% or only exceed 10% in most concentrations.

In addition, it can be seen that the sensitivity of the gas sensor 10 of the present disclosure is 20% or more even when the concentration of formaldehyde A is less than 100 ppb, but the sensitivity of the gas sensor 10 does not exceed 20% even when the concentration of each of ethanol B and toluene C is 1000 ppb or more.

When the sensing layer 300 is made of Mg-SnO₂ and the electrode 200 is made of ITO, which will be described later, the sensitivity of the gas sensor 10 to formaldehyde A is 20 to 60% when the concentration of the formaldehyde A is 0.03ppm to 0.30ppm.

However, the sensitivity of the gas sensor 10 of the present disclosure to a concentration of ethanol B of 1 ppm is 2.3%, which is very low. The sensitivity corresponds to the sensitivity of the gas sensor 10 to formaldehyde of 0.01 ppm or less.

The sensitivity of the gas sensor 10 of the present disclosure to the concentration of toluene C of 1.5ppm is measured to be 6.8%, but the sensitivity corresponds to the concentration of formaldehyde of 0.01ppm or less.

As a result, the gas sensor 10 of the present disclosure may detect, for example, formaldehyde A of a concentration near the concentration of 0.08ppm, which is the environmental standard of the International Health Organization, with high sensitivity. In addition, it can be seen that the gas sensor 10 of the present disclosure measures a volatile organic compound including ethanol B or toluene C with sensitivity to the concentration of 0.01 ppm or less based on formaldehyde, and has selectivity to formaldehyde.

According to the present invention, the sensing layer 300 is made of the compound Mg-SnO₂ and specifically detects formaldehyde.

Meanwhile, in the gas sensor 10 of the present disclosure, other metal elements may not be applied to make the sensing layer 300. This is because it is a well-known fact that the gas sensors of a prior art apply several different metal elements as a sensing material, and thus do not have the effect of specifically detecting formaldehyde, but only detect the presence of most volatile organic compounds as a whole and concentration thereof.

Additionally, when a metal other than alkali metals or alkaline earth metals is experimentally used as a material for the sensing layer 300, it is not possible to obtain an experimental result that the sensing layer 300 specifically reacts only to formaldehyde or has a higher sensitivity to formaldehyde than ethanol or toluene as illustrated in FIG. 3.

In addition, the gas sensor 10 of the present disclosure may exclude nickel and nickel oxides from the alkali metal element. In the detection sensor of the prior art used in FIG. 1, a compound of NiO and SnO₂ is used as a material for the sensing layer. Specifically, in the detection sensor of the prior art, SnO₂-NiO is used as a material of the sensing layer, and gold (Au) is used as a material of the electrode. As illustrated in FIG. 1, when formaldehyde is 0.03ppm to 0.20ppm, the sensitivity of the detection sensor of the prior art to the formaldehyde is 26 to 61%. However, the detection sensor of the prior art has a sensitivity of 68% to the concentration of 1ppm of ethanol, which is rather higher than the sensitivity to the formaldehyde.

As a result, the detection sensor of the prior art has a greater response or sensitivity to ethanol when a nickel compound is included in a sensing material, so it can be seen that when formaldehyde and ethanol are present in the air or indoors, the sensing layer cannot distinguish the formaldehyde and the ethanol from each other. It may be understood that this is because, in the case of nickel, the molecular structures of an oxide and the diameter of an atom do not specifically react only to formaldehyde, but react more strongly to gases composed of organic compounds having other functional groups.

That is, it may be understood that nickel reacts more easily, strongly, and rapidly to ethanol than to formaldehyde. Accordingly, in the sensing layer 300 of the gas sensor 10 of the present disclosure, nickel and a nickel oxide may be excluded.

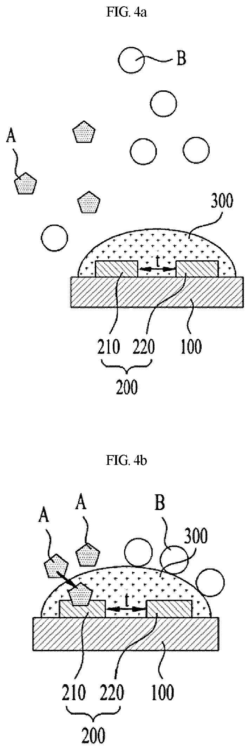

FIGS. 4a and 4b illustrate a mechanism by which the gas sensor 10 of the present disclosure specifically reacts to formaldehyde. In metal oxides, metal ions and non-metal ions combine to form a non-stoichiometric composition from a stoichiometric composition. Lattice defects accompanied in a process in which metal ions and oxide ions form a non-stoichiometric composition from a stoichiometric composition act as a donor or an acceptor.

For the Mg-SnO₂ sensing sensing layer 300 of the gas sensor 10 of the present disclosure. the formaldehyde or a formaldehyde reactor may be introduced into the lattice structure of the sensing layer 300.

The following description will be made with reference to FIG. 4a. Volatile organic compounds including formaldehyde A may exist indoors or in the air. For example, formaldehyde A and the ethanol B may coexist indoors or in the air.

FIG. 4b illustrates that formaldehyde A is accommodated in the lattice structure of the sensing layer 300. In this way, when formaldehyde A is accommodated in the lattice structure of the sensing layer 300, the capacitance or electrical resistance of the entire sensing layer 300 may be changed.

In addition, formaldehyde A accommodated in the sensing layer 300 may generate the movement of a large amount of electrons while strongly generating oxidation-reduction reactions with alkali metals or alkaline earth metals. Accordingly, the movement of a large amount of electrons generated inside the sensing layer 300 may be amplified and the electrons may be transferred to the electrode 200, and this may change the capacitance or electrical resistance of the electrode 200.

Accordingly, the gas sensor 10 of the present invention detects even small amounts of formaldehyde A with high sensitivity. However, volatile organic compounds having large molecular structures or other functional groups such as the ethanol B or toluene (C) other than formaldehyde A cannot be introduced into the lattice structure of the sensing layer 300.

In other words, although ethanol B may contact the sensing layer 300, the ethanol B cannot be introduced into the lattice structure of the inside of the sensing layer 300. Furthermore, the ethanol B cannot cause a strong redox reaction with the sensing layer 300.

Accordingly, a volatile organic compound such as ethanol B cannot generate the movement of a large amount of electrons in the sensing layer 300 and cannot change the capacitance or electrical resistance of the sensing layer 300.

As a result, the gas sensor 10 of the present disclosure may not respond or have a very low sensitivity to volatile organic compounds except for formaldehyde. Accordingly, the gas sensor 10 of the present disclosure may secure high sensitivity and detection specificity to formaldehyde A.

Meanwhile, as a result of an experiment, it can be seen that the gas sensor 10 of the present disclosure requires that the electrode 200 be made of an indium tin oxide (ITO) so that the above-mentioned effect appears or becomes larger. This means that when the electrode 200 is made of a general compound of gold (Au) or platinum (Pt), the reception sensitivity of the specific capacitance change or electron movement occurring in the sensing layer 300 may be reduced.

Specifically, the electrode 200 may be made of a metal oxide including at least one element of Ti, Zn, In, and Sn, and may not include gold (Au) or platinum (Pt).

The sensing layer 300 may be formed to completely cover the electrode 200. In this way, the first electrode 210 and the second electrode 220 may be securely connected to each other by the sensing layer 300.

In addition, the sensing layer 300 may further improve the performance of detecting formaldehyde by maximizing an area of the sensing layer 300 exposed to atmospheric or indoor air.

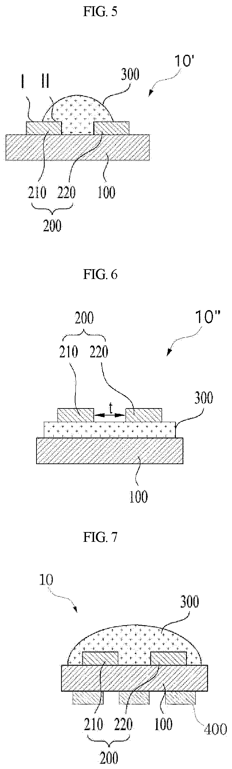

FIG. 5 illustrates another embodiment of the gas sensor 10' of the present disclosure. In the gas sensor 10' according to the embodiment, the sensing layer 300 has a property that reacts specifically to formaldehyde, so the sensing layer 300 may not be required to cover the entirety of the electrode 200.

Accordingly, in the gas sensor 10' according to the embodiment, a portion I of a surface area of the electrode 200 may be exposed to the outside, and a remaining area II thereof may be covered by the sensing layer 300. The exposed part of the electrode 200 may be referred to as the exposed area I, and the area covered by the sensing layer 300 may be referred to as the blocked area II.

In this embodiment, it is considered that the sensing layer 300 may function properly as long as the sensing layer 300 can contact formaldehyde in the air or indoors while connecting the first electrode 210 and the second electrode 220 to each other. In this way, since the sensing layer 300 can be formed in a relatively narrow area, the amount of a material of which the sensing layer 300 is formed may be minimized.

In addition, by placing the exposed area I, it is possible to easily check whether the electrode 200 is properly installed and whether the electrode 200 is operating normally. For reference, as in this embodiment, when the electrode 200 has the exposed area I, the exposed area I may be used as a part to which a lead wire for connection to the outside is connected, so the size of the gas sensor 10' may be miniaturized.

FIG. 6 illustrates still another embodiment of the gas sensor 10" of the present disclosure. In the gas sensor 10" according to the embodiment, the sensing layer 300 has a property that reacts specifically to formaldehyde A, and thus as long as the electrodes of the electrode 200 can be connected to each other by the sensing layer 300, the sensing layer 300 may function properly at any place at which the sensing layer 300 is located.

In the embodiment, the sensing layer 300 may be formed on the substrate 100, and the electrode 200 may be formed on the sensing layer 300. The electrode 200 may be formed as one pair of the first electrode 210 and the second electrode 220 spaced apart from each other by a predetermined distance t. Here, each of the first electrode 210 and the second electrode 220 may be connected to the outside by a separate lead wire (not shown).

In addition, the electrode 200 does not react to formaldehyde A or volatile organic compounds, so the electrode 200 may be exposed indoors or to the atmosphere. In consideration of this point, the electrode 200 may be formed on the sensing layer 300.

In the embodiment, the sensing layer 300 may be first formed on the substrate 100, and the electrode 200 may be formed on the sensing layer 300. That is, the sensing layer 300 may be located between the substrate 100 and the electrode 200, and the first electrode 210 and the second electrode 220 may be formed on the surface of the sensing layer 300. Accordingly, the first electrode 210 and the second electrode 220 may be formed on the sensing layer 300, and thus it may be guaranteed that the first electrode 210 and the second electrode 220 are connected to each other by the sensing layer 300.

In the embodiment, except for surfaces on which the first electrode 210 and the second electrode 220 are seated on the sensing layer 300, all outer surfaces of the first electrode 210 and the second electrode 220 may be exposed to the outside. Accordingly, it may be easier to connect a lead wire for electrical connection with the outside. Particularly, the formation of the first electrode 210 and the second electrode 220 may be relatively easy in a manufacturing process thereof, and, after the manufacturing, maintenance thereof may be relatively easy.

Next, FIG. 7 illustrates that a heater 400 is added in the embodiment illustrated in FIG. 2. Here, for convenience of explanation, the same reference numerals as in the embodiment illustrated in FIG. 2 are used, and the heater 400 is added.

The heater 400 provides heat to the gas sensor 10 so that the gas sensor 10 can operate at a specific temperature. The heater 400 may be made of gold or platinum. As illustrated in FIG. 7, the heater 400 may be located on a surface opposite to the surface of the substrate 100 on which the electrode 200 is located. However, heaters 400 may be located around the electrode 200 on the surface on which the electrode 200 is located so as to surround the electrode 200.

Hereinafter, one embodiment of the manufacturing method of the gas sensor 10 of the present disclosure will be described. For convenience of explanation, the manufacturing method will be described based on the gas sensor 10 illustrated in FIG. 2.

In the gas sensor 10 of the present disclosure, as the material of the electrode 200, an indium tin oxide (ITO) may be used. As the material of the sensing layer 300, Mg-SnO₂ is used.

The manufacturing of Mg-SnO2 will be described. Tin chloride (SnCl₂) is reacted with an aqueous solution of magnesium sulfate. Here, the ratio of the contents of Sn and Mg may be preset to be Sn:Mg = 20:1 in terms of a molar ratio. A precipitate may be obtained by dropping aqueous ammonia into this aqueous solution.

This precipitate may be washed with distilled water and then dried. The drying may be done in the air. Mg-SnO₂ may be obtained by calcining the dried powder at 600 to 800°C.

The electrode 200 made of an indium tin oxide (ITO) may be formed on the surface of the substrate 100. After forming the first electrode 210 and the second electrode 220 of the electrode 200 on the substrate 100, the sensing layer 300 may be formed. The sensing layer 300 uses the above-described Mg-SnO₂ as a material. The sensing layer 300 may be formed by dispersing and applying Mg-SnO₂ onto a mixed solution of pure water and glycerin to connect the first electrode 210 and the second electrode 220 to each other.

After forming the sensing layer 300, the sensing layer 300 may be heated at 500°C.

The measuring of response to gas by using the gas sensor 10 of the present disclosure will be described. The gas sensor 10 may be installed in a gas introduction container and exposed to gas whose concentration is adjusted. In this state, the sensitivity of the gas sensor 10 to the gas may be measured by detecting the change of a resistance value between the first electrode 210 and the second electrode 220.

By using pure air as a dilution gas, concentration-adjusted gas may be generated by a permeator and a flow rate thereof may be adjusted by a mass flow controller so that the gas can be introduced into the gas introduction container. Humidity may be generated by bubbling distilled water. Relative humidity obtained as the result of FIG. 3 may be preset to be 54%.

In the gas sensor 10, a resistance value may be obtained by applying voltage through wiring installed in the sensor element of an electrometer and measuring an electric current.

The gas sensor 10 of the present disclosure may be used in a variety of home appliances. For example, the gas sensor 10 may be installed in an air conditioner, an air purifier, a washing machine, a dryer, and a clothing management device to detect whether formaldehyde is generated and to perform necessary operations accordingly.

## Claims

1. A gas sensor (10) for detecting volatile organic compounds, **characterized in that** the gas sensor (10) comprises:
a substrate (100) made of a non-conductive material;
at least one pair of electrodes (200) located on the substrate (100) and formed of a conductive material, wherein the at least one pair of electrodes (200) is electrically connected to the outside; and
a sensing layer (300) configured to be in contact with at least a portion of the at least one pair of electrodes (200) or to cover the at least one pair of electrodes (200), the sensing layer (300) reacting to formaldehyde,
wherein the sensing layer (300) comprises Mg-SnO₂.

2. The gas sensor (10) of claim 1, **characterized in that** the at least one pair of electrodes (200) is formed of an indium tin oxide (ITO).

3. The gas sensor (10) of any one of claims 1 to 2, **characterized in that** the at least one pair of electrodes (200) comprises a first electrode (210) formed on the substrate (100), and a second electrode (220) formed on the substrate (100) by being spaced apart from the first electrode (210), and
the sensing layer (300) is disposed to connect the first electrode (210) with the second electrode (220).

4. The gas sensor (10) of claim 3, **characterized in that** the sensing layer (300) is configured to expose at least a portion of each of the first electrode (210) and the second electrode (220) to the air.

5. The gas sensor (10) of any one of claims 1 to 4, **characterized in that** a heater (400) is provided on the substrate (100), the heater (400) being located on a surface of the substrate (100) on which the at least one pair of electrodes (200) is located or on a surface opposite to the surface of the substrate (100) on which the at least one pair of electrodes (200) is located.

6. A gas sensor (10) for detecting volatile organic compounds, **characterized in that** the gas sensor (10) comprises:
a substrate (100) made of a non-conductive material;
a sensing layer (300) located on the substrate (100), the sensing layer (300) reacting to formaldehyde and the sensing layer being made of Mg-SnO₂; and
at least one pair of electrodes (200) made of a conductive material and configured to detect electron movement and capacitance inside the sensing layer (300), wherein the at least one pair of electrodes (200) is electrically connected to the outside,
wherein the at least one pair of electrodes (200) comprises a first electrode (210) formed of a conductive material, and a second electrode (220) configured to supply an electric current to the first electrode (210) or to receive an electric current from the first electrode (210), and
the sensing layer (300) connects the first electrode (210) with the second electrode (220) and is configured to expose at least a portion of each of the first electrode (210) and the second electrode (220) to the air.

7. The gas sensor (10) of claim 6, **characterized in that** the sensing layer (300) is disposed between the substrate (100) and the at least one pair of electrodes (200).

8. The gas sensor (10) of claim 7, **characterized in that** the first electrode (210) and the second electrode (220) are provided on an outer surface of the sensing layer (300).

9. The gas sensor (10) of claim 6, 7, or 8, **characterized in that** the sensing layer (300) shields a portion of the first electrode (210) and a portion of the second electrode (220).

10. The gas sensor (10) of any one of claims 6 to 9, **characterized in that** a heater (400) is provided on the substrate (100), the heater (400) being located on a surface of the substrate (100) on which the at least one pair of electrodes (200) is located or on a surface opposite to the surface of the substrate (100) on which the at least one pair of electrodes (200) is located.

11. A method for manufacturing a gas sensor (10) for detecting volatile organic compounds, with the gas sensor (10) having a sensing layer (300) configured to connect electrodes (200) to each other formed on a substrate (100), **characterized in that** the method comprises:
forming the electrodes (200) by using an indium tin oxide (ITO), and
forming the sensing layer (300) by using Mg-SnO₂.

12. The method of claim 11, **characterized in that** the sensing layer (300) is formed by dispersing and applying Mg-SnO₂ onto a mixed solution of pure water and glycerin and heating the mixed solution, onto which the Mg-SnO₂ is applied, to a predetermined temperature.

## Patentansprüche

1. Gassensor (10) zum Erfassen flüchtiger organischer Verbindungen, **dadurch gekennzeichnet, dass** der Gassensor (10) umfasst:
ein Substrat (100), das aus einem nichtleitenden Material hergestellt ist;
zumindest ein Elektrodenpaar (200), das auf dem Substrat (100) angeordnet und aus einem leitenden Material ausgebildet ist, wobei das zumindest eine Elektrodenpaar (200) elektrisch nach außen verbunden ist; und
eine Sensorschicht (300), die dazu eingerichtet ist, mit zumindest einem Teil des zumindest einen Elektrodenpaars (200) in Kontakt zu sein oder das zumindest eine Elektrodenpaar (200) zu bedecken, wobei die Sensorschicht (300) auf Formaldehyd reagiert,
wobei die Sensorschicht (300) Mg-SnO₂ umfasst.

2. Gassensor (10) nach Anspruch 1, **dadurch gekennzeichnet, dass** das zumindest eine Elektrodenpaar (200) aus einem Indiumzinnoxid (ITO) ausgebildet ist.

3. Gassensor (10) nach einem der Ansprüche 1 bis 2, **dadurch gekennzeichnet, dass** das zumindest eine Elektrodenpaar (200) eine erste Elektrode (210), die auf dem Substrat (100) ausgebildet ist, und eine zweite Elektrode (220), die auf dem Substrat (100) von der ersten Elektrode (210) beabstandet ausgebildet ist, umfasst, und
die Sensorschicht (300) angeordnet ist, um die erste Elektrode (210) mit der zweiten Elektrode (220) zu verbinden.

4. Gassensor (10) nach Anspruch 3, **dadurch gekennzeichnet, dass** die Sensorschicht (300) dazu eingerichtet ist, jeweils zumindest einen Teil der ersten Elektrode (210) und der zweiten Elektrode (220) der Luft auszusetzen.

5. Gassensor (10) nach einem der Ansprüche 1 bis 4, **dadurch gekennzeichnet, dass** ein Heizelement (400) auf dem Substrat (100) vorgesehen ist, wobei das Heizelement (400) auf einer Oberfläche des Substrats (100), auf der das zumindest eine Elektrodenpaar (200) angeordnet ist, oder auf einer Oberfläche angeordnet ist, die der Oberfläche des Substrats (100), auf der das zumindest eine Elektrodenpaar (200) angeordnet ist, gegenüberliegt.

6. Gassensor (10) zum Erfassen flüchtiger organischer Verbindungen, **dadurch gekennzeichnet, dass** der Gassensor (10) umfasst:
ein Substrat (100), das aus einem nichtleitenden Material hergestellt ist;
eine Sensorschicht (300), die auf dem Substrat (100) angeordnet ist, wobei die Sensorschicht (300) auf Formaldehyd reagiert und die Sensorschicht aus Mg-SnO₂ hergestellt ist; und
zumindest ein Elektrodenpaar (200), das aus einem leitenden Material hergestellt und dazu eingerichtet ist, Elektronenbewegung und Kapazität innerhalb der Sensorschicht (300) zu erfassen, wobei das zumindest eine Elektrodenpaar (200) elektrisch nach außen verbunden ist,
wobei das zumindest eine Elektrodenpaar (200) eine erste Elektrode (210), die aus einem leitenden Material ausgebildet ist, und eine zweite Elektrode (220), die dazu eingerichtet ist, der ersten Elektrode (210) einen elektrischen Strom zuzuführen oder von der ersten Elektrode (210) einen elektrischen Strom zu empfangen, umfasst, und
die Sensorschicht (300) die erste Elektrode (210) mit der zweiten Elektrode (220) verbindet und dazu eingerichtet ist, jeweils zumindest einen Teil der ersten Elektrode (210) und der zweiten Elektrode (220) der Luft auszusetzen.

7. Gassensor (10) nach Anspruch 6, **dadurch gekennzeichnet, dass** die Sensorschicht (300) zwischen dem Substrat (100) und dem zumindest einen Elektrodenpaar (200) angeordnet ist.

8. Gassensor (10) nach Anspruch 7, **dadurch gekennzeichnet, dass** die erste Elektrode (210) und die zweite Elektrode (220) auf einer Außenfläche der Sensorschicht (300) vorgesehen sind.

9. Gassensor (10) nach Anspruch 6, 7 oder 8, **dadurch gekennzeichnet, dass** die Sensorschicht (300) einen Teil der ersten Elektrode (210) und einen Teil der zweiten Elektrode (220) abschirmt.

10. Gassensor (10) nach einem der Ansprüche 6 bis 9, **dadurch gekennzeichnet, dass** ein Heizelement (400) auf dem Substrat (100) vorgesehen ist, wobei das Heizelement (400) auf einer Oberfläche des Substrats (100), auf der das zumindest eine Elektrodenpaar (200) angeordnet ist, oder auf einer Oberfläche angeordnet ist, die der Oberfläche des Substrats (100), auf der das zumindest eine Elektrodenpaar (200) angeordnet ist, gegenüberliegt.

11. Verfahren zum Herstellen eines Gassensors (10) zum Erfassen flüchtiger organischer Verbindungen, wobei der Gassensor (10) eine auf einem Substrat (100) ausgebildete Sensorschicht (300) aufweist, die dazu eingerichtet ist, Elektroden (200) miteinander zu verbinden, **dadurch gekennzeichnet, dass** das Verfahren umfasst:
Ausbilden der Elektroden (200) unter Verwendung eines Indiumzinnoxids (ITO), und
Ausbilden der Sensorschicht (300) unter Verwendung von Mg-SnO₂.

12. Verfahren nach Anspruch 11, **dadurch gekennzeichnet, dass** die Sensorschicht (300) durch Dispergieren und Aufbringen von Mg-SnO₂ auf eine Mischlösung aus reinem Wasser und Glycerin und Erwärmen der Mischlösung, auf die das Mg-SnO₂ aufgebracht wird, auf eine vorbestimmte Temperatur ausgebildet wird.

## Revendications

1. Capteur de gaz (10) pour détecter des composés organiques volatils, **caractérisé en ce que** le capteur de gaz (10) comprend :
un substrat (100) constitué d'un matériau non conducteur ;
au moins une paire d'électrodes (200) située sur le substrat (100) et formée d'un matériau conducteur, ladite au moins une paire d'électrodes (200) étant électriquement reliée à l'extérieur ; et
une couche de détection (300) configurée pour être en contact avec au moins une partie de ladite au moins une paire d'électrodes (200) ou pour recouvrir ladite au moins une paire d'électrodes (200), la couche de détection (300) réagissant au formaldéhyde,
dans lequel la couche de détection (300) comprend du Mg-SnO₂.

2. Capteur de gaz (10) selon la revendication 1, **caractérisé en ce que** ladite au moins une paire d'électrodes (200) est formée d'un oxyde d'indium-étain (ITO).

3. Capteur de gaz (10) selon l'une quelconque des revendications 1 à 2, **caractérisé en ce que** ladite au moins une paire d'électrodes (200) comprend une première électrode (210) formée sur le substrat (100) et une seconde électrode (220) formée sur le substrat (100) en étant espacée de la première électrode (210), et
la couche de détection (300) est disposée pour relier la première électrode (210) à la seconde électrode (220).

4. Capteur de gaz (10) selon la revendication 3, **caractérisé en ce que** la couche de détection (300) est configurée pour exposer à l'air au moins une partie de chacune de la première électrode (210) et de la seconde électrode (220).

5. Capteur de gaz (10) selon l'une quelconque des revendications 1 à 4, **caractérisé en ce qu'**un élément chauffant (400) est prévu sur le substrat (100), l'élément chauffant (400) étant situé sur une surface du substrat (100) sur laquelle ladite au moins une paire d'électrodes (200) est située ou sur une surface opposée à la surface du substrat (100) sur laquelle ladite au moins une paire d'électrodes (200) est située.

6. Capteur de gaz (10) pour détecter des composés organiques volatils, **caractérisé en ce que** le capteur de gaz (10) comprend :
un substrat (100) constitué d'un matériau non conducteur ;
une couche de détection (300) située sur le substrat (100), la couche de détection (300) réagissant au formaldéhyde et la couche de détection étant constituée de Mg-SnO₂ ; et
au moins une paire d'électrodes (200) constituée d'un matériau conducteur et configurée pour détecter le mouvement des électrons et la capacité électrique à l'intérieur de la couche de détection (300), ladite au moins une paire d'électrodes (200) étant électriquement reliée à l'extérieur,
dans lequel ladite au moins une paire d'électrodes (200) comprend une première électrode (210) formée d'un matériau conducteur et une seconde électrode (220) configurée pour fournir un courant électrique à la première électrode (210) ou pour recevoir un courant électrique de la première électrode (210), et
la couche de détection (300) relie la première électrode (210) à la seconde électrode (220) et est configurée pour exposer à l'air au moins une partie de chacune de la première électrode (210) et de la seconde électrode (220).

7. Capteur de gaz (10) selon la revendication 6, **caractérisé en ce que** la couche de détection (300) est disposée entre le substrat (100) et ladite au moins une paire d'électrodes (200).

8. Capteur de gaz (10) selon la revendication 7, **caractérisé en ce que** la première électrode (210) et la seconde électrode (220) sont prévues sur une surface extérieure de la couche de détection (300).

9. Capteur de gaz (10) selon la revendication 6, 7 ou 8, **caractérisé en ce que** la couche de détection (300) protège une partie de la première électrode (210) et une partie de la seconde électrode (220).

10. Capteur de gaz (10) selon l'une quelconque des revendications 6 à 9, **caractérisé en ce qu'**un élément chauffant (400) est prévu sur le substrat (100), l'élément chauffant (400) étant situé sur une surface du substrat (100) sur laquelle ladite au moins une paire d'électrodes (200) est située ou sur une surface opposée à la surface du substrat (100) sur laquelle ladite au moins une paire d'électrodes (200) est située.

11. Procédé de fabrication d'un capteur de gaz (10) pour détecter des composés organiques volatils, le capteur de gaz (10) ayant une couche de détection (300) formée sur un substrat (100) et configurée pour relier des électrodes (200) entre elles, **caractérisé en ce que** le procédé comprend :
la formation des électrodes (200) par utilisation d'un oxyde d'indium-étain (ITO), et
la formation de la couche de détection (300) par utilisation de Mg-SnO₂.

12. Procédé selon la revendication 11, **caractérisé en ce que** la couche de détection (300) est formée par dispersion et application de Mg-SnO₂ sur une solution mixte d'eau pure et de glycérine et par chauffage de la solution mixte, sur laquelle le Mg-SnO₂ est appliqué, à une température prédéterminée.
